# EUROPÄISCHE PATENTSCHRIFT

(11) **EP 3 598 948 B1**
(45) Veröffentlichungstag und Bekanntmachung des Hinweises auf die Patenterteilung: **16.03.2022**
(21) Anmeldenummer: 18186096.6
(22) Anmeldetag: 27.07.2018
(51) Int. Cl.: A61B 6/00, A61B 6/12, A61B 1/00, A61B 1/005, A61B 1/018

(54) **BILDGEBENDES SYSTEM UND VERFAHREN ZUM ERZEUGEN EINER STEREOSKOPISCHEN DARSTELLUNG, COMPUTERPROGRAMM UND DATENSPEICHER**
IMAGING SYSTEM AND METHOD FOR GENERATING A STEREOSCOPIC REPRESENTATION, COMPUTER PROGRAM AND DATA MEMORY
SYSTÈME D'IMAGERIE ET PROCÉDÉ DE GÉNÉRATION D'UNE REPRÉSENTATION STÉRÉOSCOPIQUE, PROGRAMME INFORMATIQUE ET MÉMOIRE DE DONNÉES

(43) Veröffentlichungstag der Anmeldung: 29.01.2020
(73) Patentinhaber: Siemens Healthcare GmbH, 91052 Erlangen (DE)
(72) Erfinder: Regensburger, Alois, 91058 Erlangen (DE); Alzaga, Amilcar, 90453 Nürnberg (DE)

(56) Entgegenhaltungen:
- WO-A1-2015/023990
- US-A1- 2013 218 024
- O. Kutter and A. Aichert and C. Bichlmeier and Christoph Bichlmeier and S. M. Heining and B. Ockert and E. Euler and N. Navab: "Real-time Volume Rendering for High Quality Visualization in Augmented Reality", International Workshop on Augmented environments for Medical Imaging including Augmented Reality in Computer-aided Surgery (AMI-ARCS 2008), 1. September 2008 (2008-09-01), Seite 10 pp., XP055527958, USA Gefunden im Internet: URL:http://campar.in.tum.de/pub/kutter2008 amiarcs/kutter2008amiarcs.pdf [gefunden am 2019-07-08]

## Beschreibung

Die Erfindung betrifft ein bildgebendes System zum Erzeugen einer stereoskopischen Darstellung eines Zielobjekts und ein entsprechendes Verfahren zum Erzeugen einer stereoskopischen Darstellung eines Zielobjekts. Die Erfindung betrifft weiterhin ein entsprechendes Computerprogramm oder Computerprogrammprodukt sowie einen entsprechenden Datenspeicher.

Für verschiedenste Zwecke und in verschiedensten Anwendungsbereichen werden unterschiedlichste Arten von Objekten untersucht und/oder manipuliert. Dabei werden die jeweiligen Objekte oftmals mittels einer Kamera abgebildet und ein entsprechendes Kamerabild des Objekts wird auf einer Anzeigeeinrichtung dargestellt. Dies kann beispielsweise dann besonders vorteilhaft sein, wenn ein zu untersuchender Bereich des Objekts nicht ohne weiteres zugänglich oder einsehbar ist, beispielsweise aufgrund beschränkter Platzverhältnisse und/oder aufgrund von für eine direkte Beobachtung unzureichenden Lichtverhältnissen. Die heutzutage verfügbare Kameratechnik erlaubt es auch in derartigen Situationen nutzbringend verwertbare Bilder zu erzeugen. In beengten Platzverhältnissen kann beispielsweise eine miniaturisierte Endoskopkamera eingesetzt werden. Damit können beispielsweise Maschinenanlagen oder Industrieerzeugnisse ebenso abgebildet werden wie beispielsweise Teile von Bauwerken oder Infrastruktureinrichtungen aber auch beispielsweise natürliche Objekte wie etwa Pflanzen, Tiere und Menschen oder biologische Gewebeproben. Bei der Verwendung einer optischen Kamera besteht dabei die Problematik, dass viele Materialien optisch undurchsichtig sind und daher aus Blickrichtung der Kamera hinter einem solchen undurchsichtigen Material angeordnete Bereiche nicht oder zumindest nicht ohne bereichsweise Zerstörung oder Verletzung des Materials mittels der Kamera abgebildet werden können. Wird anstelle der Kamera beispielsweise ein Röntgengerät verwendet, so können zwar optisch undurchsichtige Materialien durchdrungen werden, entsprechende Röntgenbilder sind jedoch für einen jeweiligen Benutzer oftmals weniger anschaulich und/oder aufgrund der inhärenten Eigenschaften der Röntgentechnik weniger detailreich oder weniger aussagekräftig, da für die Röntgenstrahlung zumindest im Wesentlichen transparente Materialien nicht abgebildet werden. Eine weitere wesentliche Problematik besteht darin, dass oftmals nur 2D Kamerabilder aufgenommen werden oder zur Verfügung stehen und somit auch bei einer Einblendung von unterstützenden Pfeilen, Hilfslinien, Konturlinien oder dergleichen keine definierte oder verlässliche Tiefeninformation oder Tiefenwirkung in entsprechenden Darstellungen vorhanden ist. Hierdurch wird eine zielgenaue Orientierung und Untersuchung des jeweiligen Objekts oftmals deutlich erschwert.

In der Veröffentlichung 0. Kutter et al., "Real-time Volume Rendering for High Quality Visualization in Augmented Reality", International Workshop on Augmented environments for Medical Imaging including Augmented Reality in Computer-aided Surgery (AMI-ARCS 2008) wird ein Ansatz beschrieben, der eine stereografische Visualisierung von CT-Volumendaten in einem Head-mounted Display, HMD, erlaubt, wobei volumetrische CT-Daten einer Kameraaufnahme überlagert werden. Es sind zwei Tracking-Systeme vorgesehen, die jeweils ein Infrarotkamerasystem enthalten. Ein Infrarotkamerasystem ist dabei an dem HMD selbst montiert, das andere an der Decke des Raums in dem sich der Benutzer befindet. Die CT-Daten werden dann mit dem Koordinatensystem eines der Tracking-Systeme registriert. Sowohl mittels CT als auch mittels der Tracking-Systeme werden beispielsweise Marker erfasst, die die korrekte Positionierung der angezeigten Volumendaten ermöglichen.

Aufgabe der vorliegenden Erfindung ist es, eine verbesserte Abbildung eines zumindest teilweise optisch undurchsichtigen Zielobjekts zu ermöglichen. Diese Aufgabe wird erfindungsgemäß durch die Gegenstände der unabhängigen Patentansprüche gelöst. Vorteilhafte Weiterbildungen und Ausgestaltungen der vorliegenden Erfindung sind in den abhängigen Patentansprüchen, in der Beschreibung und in den Figuren angegeben.

Ein erfindungsgemäßes bildgebendes System dient, ist also eingerichtet, zum Erzeugen einer stereoskopischen Darstellung eines Zielobjekts und weist dazu zumindest eine Erfassungseinrichtung und eine Datenverarbeitungseinrichtung auf. Die Erfassungseinrichtung ist dazu eingerichtet, ein optisches Kamerabild eines optisch undurchsichtigen Teils des Zielobjekts zu erfassen. Weiter ist die Erfassungseinrichtung dazu eingerichtet, mittels einer materialdurchdringenden Erfassungsmodalität eine in einer Aufnahmerichtung des Kamerabildes durch den undurchsichtigen Teil optisch verdeckte, also hinter dem undurchsichtigen Teil angeordnete, Struktur zu erfassen. Die Erfassungseinrichtung kann also insbesondere eine Kamera zum Aufnehmen des Kamerabildes umfassen. Weiter kann die Erfassungseinrichtung beispielsweise ein Röntgen- oder MRT-Gerät als die materialdurchdringende Erfassungsmodalität umfassen. Die materialdurchdringende Erfassungsmodalität kann aber zusätzlich oder alternativ ebenso eine Nachverfolgungseinrichtung, beispielsweise für ein elektromagnetisches Nachverfolgen (englisch: "Tracking"), umfassen. In diesem Fall kann das Erfassen der Struktur ein Erfassen und/oder Nachverfolgen einer Position und Lage, also Pose, der Struktur bedeuten oder umfassen, wozu die Struktur insbesondere einen entsprechenden Marker, beispielsweise eine elektromagnetische Sendespule, aufweisen kann. Ebenso kann das Erfassen im Sinne der vorliegenden Erfindung bedeuten, dass entsprechende Bild- und/oder Erfassungsdaten, also das Kamerabild und/oder die erfasste Struktur repräsentierende Daten, durch die Erfassungseinrichtung von einem bereitgestellten Datenspeicher oder einer bereitgestellten entsprechenden Datenquelle abgerufen werden.

Der optisch undurchsichtige Teil des Zielobjekts kann beispielsweise eine Wand oder Wandteil oder eine Oberfläche des Zielobjekts sein. Je nach Art des Zielobjekts kann dies beispielsweise eine Gehäuse- oder Zwischenwand, eine Gewebewand, eine Außenseite, eine Organoberfläche oder dergleichen mehr sein. Die optisch verdeckte Struktur kann ein innerer Teil des Zielobjekts selbst aber ebenso beispielsweise ein in oder an dem Zielobjekt angeordnetes Fremdobjekt, beispielsweise ein Fremdkörper, ein Instrument oder ein Werkzeug oder dergleichen sein oder ein solches umfassen.

Die Datenverarbeitungseinrichtung des erfindungsgemäßen bildgebenden Systems ist dazu eingerichtet, automatisch einen in dem Kamerabild und mittels der materialdurchdringenden Erfassungsmodalität abgebildeten gemeinsamen Referenzpunkt zu bestimmen. Dieser Referenzpunkt kann also ein letztlich irgendwie ausgearteter Punkt sein, der sowohl in dem Kamerabild abgebildet und erkennbar als auch in den mittels der materialdurchdringenden Erfassungsmodalität erfassten oder erzeugten oder von dieser bereitgestellten Erfassungsdaten identifizierbar ist. Der Referenzpunkt kann beispielsweise automatisch mittels eines Objekterkennungs- und/oder Bildverarbeitungsalgorithmus bestimmt werden. Dazu kann es ebenfalls vorgesehen sein, das Kamerabild mit dem Erfassungsdaten der materialdurchdringenden Erfassungsmodalität zu registrieren, wofür bekannte Methoden verwendet werden können. Nach dem Registrieren kann dann ein gemeinsamer Punkt, also ein Berührungspunkt jeweiliger erfasster Bereiche des Zielobjekts als der Referenzpunkt bestimmt werden.

Weiter ist die Datenverarbeitungseinrichtung dazu eingerichtet, automatisch eine stereoskopische Tiefenlage, also eine Vergenz oder Parallaxe, des Referenzpunktes auf einen vorgegebenen Wert festzulegen. Beispielsweise kann hier eine Vergenz von 0 vorgegeben sein, sodass die stereoskopische Tiefenlage des Referenzpunktes in der letztendlich erzeugten stereoskopischen Darstellung in einer Bildschirm- oder Anzeigeebene liegt. Ebenso kann beispielsweise ein bestimmter virtueller Abstand vorgegeben sein, in welchem sich in der letztendlich erzeugten stereoskopischen Darstellung der Referenzpunkt zu der Bildschirm- oder Anzeigeebene befinden, also von der Bildschirm- oder Anzeigeebene entfernt erscheinen soll. Besonders bevorzugt ist der Referenzpunkt Teil des undurchsichtigen Teils des Zielobjekts, liegt also auf oder in dessen durch das Kamerabild abgebildeter Oberfläche.

Weiter ist die Datenverarbeitungseinrichtung dazu eingerichtet, anhand der erfassten verdeckten Struktur automatisch oder teilautomatisch ein Überlagerungsbild zu erzeugen oder zu erfassen. Dieses Überlagerungsbild kann im einfachsten Fall ein mittels der materialdurchdringenden Erfassungsmodalität erfasstes oder erzeugtes Bild der Struktur sein. Ebenso kann das Überlagerungsbild aus den Erfassungsdaten der materialdurchdringenden Erfassungsmodalität beispielsweise durch Drehen, Verschieben und/oder Verzerren erzeugt werden, um eine Darstellung oder Perspektive des Überlagerungsbildes an die Aufnahmerichtung, also eine Perspektive des Kamerabildes anzupassen. Das Überlagerungsbild kann ebenso ein aus den Erfassungsdaten erzeugtes Modell der Struktur und/oder wenigstens ein virtuelles Objekt sein oder umfassen, also darstellen. Das virtuelle Objekt oder die virtuelle Darstellung kann beispielsweise eine Markierung eines Raumbereiches oder einer Fläche, eine Hilfslinie oder dergleichen sein oder umfassen. Das wenigstens eine virtuelle Objekt und/oder das erzeugte Modell kann zweidimensional oder - bevorzugt -dreidimensional sein.

Weiter ist die Datenverarbeitungseinrichtung dazu eingerichtet, automatisch die stereoskopische Darstellung aus dem Kamerabild und dem Überlagerungsbild zu erzeugen und dabei automatisch eine stereoskopische Tiefenlage des Kamerabildes auf die stereoskopische Tiefenlage des Referenzpunktes festzulegen und in Abhängigkeit von dieser eine stereoskopische Tiefenlage des Überlagerungsbildes in Bezug auf die stereoskopische Tiefenlage des Referenzpunktes einzustellen, sodass in der stereoskopischen Darstellung das Überlagerungsbild zumindest in oder an dem Referenzpunkt in Aufnahmerichtung des Kamerabildes vor und/oder hinter dem optisch undurchsichtigen Teil erscheint gemäß einer entsprechenden realen Lage vor und/oder hinter dem optisch undurchsichtigen Teil. Ist oder umfasst das Überlagerungsbild ein Modell der verdeckten Struktur, so wird dieses Modell also in der stereoskopischen Darstellung entsprechend hinter dem optische undurchsichtigen Teil dargestellt. Ist oder umfasst das Überlagerungsbild hingegen ein virtuelles Objekt, welches nicht notwendigerweise eine real vorhandene physische Entsprechung haben muss, so kann dieses Objekt vor und/oder hinter dem optisch undurchsichtigen Teil dargestellt werden, je nachdem, ob bei einer Übertragung in die Realität eine Entsprechung oder Repräsentation des virtuellen Objekts vor und/oder hinter dem optisch undurchsichtigen Teil angeordnet wäre. Es ist dabei vorliegend aber vorgesehen, dass diese Lage- oder Tiefenbeziehungen in dem Referenzpunkt gültig sind. In anderen Punkten oder Bereichen der stereoskopischen Darstellung können Inkonsistenzen der Tiefenlage in der stereoskopischen Darstellung in Kauf genommen werden. Letzteres kann beispielsweise der Fall sein oder auftreten, wenn der optisch undurchsichtige Teil durch das Kamerabild zweidimensional abgebildet wird, real aber räumlich gewölbt oder gebogen ist.

Die Tiefenlagen des Kamerabildes und des Überlagerungsbildes oder deren Tiefenbeziehungen in der stereoskopischen Darstellung sind hier also explizit auf den Referenzpunkt bezogen. In dem Referenzpunkt entsprechen die Tiefenlagen oder Tiefenbeziehungen daher den entsprechenden realen räumlichen Verhältnissen. Dies muss jedoch in anderen Punkten oder Raumbereichen nicht notwendigerweise der Fall sein. Die Tiefenbeziehungen, also die relativen stereoskopischen Tiefenlagen, können in anderen Punkten oder Bereichen außer dem Referenzpunkt zwar bevorzugt korrekt sein, also den entsprechenden realen räumlichen Verhältnissen entsprechen, bezüglicher einer jeweiligen Tiefenlage vor und/oder hinter dem optisch undurchsichtigen Teil. Dabei müssen jedoch insbesondere absolute Abstände oder Entfernungen oder absolute Entfernungsverhältnisse in Tiefenrichtung nicht den entsprechenden realen absoluten Abständen oder Entfernungen oder absoluten Entfernungsverhältnissen entsprechen. Dadurch kann hier also vorteilhaft eine entsprechende, mit erheblichem Aufwand verbundene räumliche Vermessung entfallen und dennoch einem Betrachter ein gegenüber einer nicht-stereoskopischen Überlagerung ein verbesserter räumlicher Tiefeneindruck vermittelt werden.

Da der Referenzpunkt gerade die einzige für den Betrachter relevante Verbindung zwischen dem optisch undurchsichtigen Teil, also der in dem oder durch das Kamerabild abgebildeten sichtbaren Oberfläche, und der dahinter liegender verdeckten beziehungsweise der entsprechenden virtuellen Struktur ist, reicht es hier aus, dass die Tiefenbeziehung an dem Referenzpunkt sinnvoll und korrekt ist, also dort den realen räumlichen Verhältnissen entspricht.

Ist also beispielsweise das Überlagerungsbild ein Modell der optisch verdeckten Struktur, so wird diese in der stereoskopischen Darstellung dann in deren vorgesehener Betrachtungsrichtung hinter dem Referenzpunkt, also - zumindest virtuell oder stereoskopisch - weiter von der Bildschirm- oder Anzeigeebene entfernt, also beispielsweise mit kleinerer Parallaxe, als der Referenzpunkt und das Kamerabild beziehungsweise zu dem Kamerabild gehörende oder aus dem Kamerabild stammende Bildteile oder Bildbereiche dargestellt. Bevorzugt kann es dann vorgesehen sein, dass keine virtuellen Strukturen eingeblendet, also in der stereoskopischen Darstellung dargestellt, werden, welche sich vor der stereoskopischen Tiefenlage des optisch aufgenommenen Kamerabildes befinden.

Umfasst das Kamerabild hingegen beispielsweise eine virtuelle Hilfslinie, so kann diese sich in der stereoskopischen Darstellung in Tiefenrichtung, also von einem Bildvordergrund in Richtung eines Bildhintergrundes erstrecken. Dann kann diese Hilfslinie, also das Überlagerungsbild, beispielsweise ausgehend von einem Punkt oder Raumbereich, welcher in der stereoskopischen Darstellung vor dem Referenzpunkt liegt, den undurchsichtigen Teil durchtreten und sich hinter dem undurchsichtigen Teil fortsetzen. Dass das Überlagerungsbild anhand der optisch verdeckten Struktur erzeugt wird, kann dann beispielsweise bedeuten, dass die Hilfslinie auf die Struktur zu führt, also beispielsweise an der Struktur endet oder beispielsweise in einem vorgegebenen Mindestabstand an der Struktur vorbeiführt, um eine Sicherheitszone zu definieren und eine Beschädigung der Struktur zu vermeiden. Das Überlagerungsbild bildet also die Struktur selbst ab und/oder steht in einer bestimmten, insbesondere vorgegebenen, räumlichen Lagebeziehung zu der Struktur. Besonders bevorzugt kann das Überlagerungsbild also ein 3D-Bild oder ein 3D-Datensatz sein. Ebenso können die mittels der materialdurchdringenden Erfassungs- oder Bildgebungsmodalität erzeugten oder erfasst Erfassungsdaten ein 3D-Bild oder ein 3D-Datensatz sein.

Die erzeugte stereoskopische Darstellung ist also ein zusammengesetztes stereoskopisches Bild mit einer Tiefenwirkung und kombiniert das Kamerabild mit dem Überlagerungsbild, also einer Einblendung. Das optische Kamerabild selbst liefert für sich genommen keine Informationen über die verdeckte Struktur, also einen Raumbereich der aus Sicht der Kamera hinter dem undurchsichtigen Teil des Zielobjekts liegt. Ein, beispielsweise aus einer seitlichen Richtung, aufgenommenes Röntgenbild kann eine rudimentäre Orientierung in diesem nicht durch die Kamera beziehungsweise das Kamerabild abgebildeten Raumbereich ermöglichen. Für eine präzise Orientierung, beispielsweise zum Steuern eines Instruments oder Werkzeugs in diesem Raumbereich, ist dies jedoch nicht optimal, da ein Blick auf derartig unterschiedliche Darstellungen wie ein Kamerabild und ein Röntgenbild sowie eine Refokussierung auf eine jeweilige Anzeigeeinrichtung von einem Benutzer oder Betrachter ein besonders gutes räumliches Vorstellungsvermögen ebenso wie geistige Rüstzeiten verlangt. Bekannte Methoden sehen beispielsweise ein Einblenden von 2D Informationen, wie beispielsweise Pfeilen oder Konturlinien auf dem monoskopischen Kamerabild vor, wodurch jedoch bei einem Blick auf dieses 2D-Kamerabild keinerlei Tiefeninformationen geliefert oder vermittelt werden, sodass eine präzise Orientierung oder Navigation kaum möglich ist.

Die vorliegende Erfindung bietet demgegenüber die Vorteile, dass sämtliche relevanten Informationen oder Daten in einem einzigen Bild, nämlich in der stereoskopischen Darstellung, vereint sind und dabei eine sinnvolle, realistische Tiefen-Lagebeziehung zueinander aufweisen. Eine besondere Schwierigkeit kann darin bestehen, dass der durch das Kamerabild abgebildete optisch undurchsichtige Teil des Zielobjekts seinerseits gewölbt sein, also eine Ausdehnung in Blickrichtung oder Aufnahmerichtung des Kamerabildes, also in der stereoskopischen Tiefenrichtung aufweisen kann. Dennoch wird dieser undurchsichtige Teil in dem 2D-Kamerabild flach dargestellt. Würden nun einfach das Überlagerungsbild, also eine räumlich in Tiefenrichtung ausgedehnte virtuelle 3D-Struktur oder - Einblendung in dem 2D-Kamerabild überlagert oder eingeblendet, so hätte das Überlagerungsbild, also die eingeblendeten 3D-Strukturen eine unbestimmte Tiefen-Lagebeziehung zu dem 2D-Kamerabild, also zu dem durch dieses abgebildeten undurchsichtigen Teil. Insbesondere kann diese unbestimmte Tiefenbeziehung, also räumliche Lagebeziehung in Tiefenrichtung, nicht überall in dem resultierenden Gesamtbild beziehungsweise nicht überall in dem Kamerabild, also nicht in allen Bereichen des räumlich in der Tiefenrichtung ausgedehnten undurchsichtigen Teils richtig, also korrekt oder realistisch sein.

Diese Schwierigkeit wird durch die vorliegende Erfindung gelöst, indem die Tiefenlage des Kamerabildes, also des abgebildeten undurchsichtigen Teils auf die Tiefenlage des Referenzpunktes festgelegt wird, wodurch eine sinnvolle und konsistente Tiefen-Lagebeziehung in der stereoskopischen Darstellung ermöglicht und erreicht wird. Es wird also die Tiefenlage des Referenzpunktes als Bezugspunkt gewählt. Da der Referenzpunkt nicht nur in dem Kamerabild, sondern auch in den mittels der materialdurchdringenden Erfassungsmodalität gewonnenen Erfassungsdaten identifizierbar ist, kann eine Tiefenlage dieses Punktes in den Erfassungsdaten auf dieselbe stereoskopische Tiefenlage, also etwa dieselbe Vergenz oder Parallaxe, festgelegt werden, in welcher der Referenzpunkt und das Kamerabild in der stereoskopischen Darstellung dargestellt werden. Der Referenzpunkt dient also als Tiefenreferenz zwischen den beiden unterschiedlichen Bildern oder Datensätzen, also dem Kamerabild und den Erfassungsdaten der materialdurchdringenden Erfassungsmodalität. Durch die vorliegende Erfindung wird also eine erweiterte Visualisierung ermöglicht, welche eine besonders präzise und effiziente Orientierung und Navigation ermöglicht, in dem in einer einzigen stereoskopischen Darstellung alle verfügbaren Daten mit einer geeigneten, insbesondere realistischen, Tiefen-Lagebeziehung zueinander kombiniert dargestellt werden.

Das erfindungsgemäße bildgebende System kann bevorzugt zudem eine mit der Datenverarbeitungseinrichtung verbundene Anzeigeeinrichtung zum Anzeigen der stereoskopischen Darstellung umfassen. Diese Anzeigeeinrichtung kann also insbesondere eine Stereo-Anzeigeeinrichtung, wie beispielsweise ein 3D-Bildschirm oder eine geeignete Stereo-Datenbrille oder dergleichen sein.

Ein erfindungsgemäßes Verfahren dient zum Erzeugen einer stereoskopischen Darstellung eines Zielobjekts mittels eines bildgebenden Systems, insbesondere mittels des erfindungsgemäßen bildgebenden Systems. In einem Verfahrensschritt des erfindungsgemäßen Verfahrens wird ein optisches Kamerabild eines optisch undurchsichtigen Teils des Zielobjekts mittels einer entsprechenden Erfassungseinrichtung erfasst. In einem weiteren Verfahrensschritt wird mittels einer materialdurchdringenden Erfassungsmodalität eine in einer Aufnahmerichtung des optischen Kamerabildes durch den undurchsichtigen Teil optisch verdeckte Struktur erfasst. Dies kann mittels einer separaten oder mittels derselben Erfassungseinrichtung erfolgen, mittels welcher das optische Kamerabild erfasst wird. In einem weiteren Verfahrensschritt wird automatisch ein in dem Kamerabild und mittels der materialdurchdringenden Erfassungsmodalität, also in entsprechenden Erfassungsdaten, abgebildeter gemeinsamer Referenzpunkt mittels einer entsprechend eingerichteten Datenverarbeitungseinrichtung bestimmt. In einem weiteren Verfahrensschritt wird ebenfalls mittels der oder einer Datenverarbeitungseinrichtung automatisch eine stereoskopische Tiefenlage des Referenzpunktes auf einen vorgegebenen Wert festgelegt. In einem weiteren Verfahrensschritt wird ebenfalls mittels der oder einer Datenverarbeitungseinrichtung automatisch anhand der erfassten verdeckten Struktur ein Überlagerungsbild erzeugt. In einem weiteren Verfahrensschritt wird ebenfalls mittels der oder einer Datenverarbeitungseinrichtung automatisch die stereoskopische Darstellung des Zielobjekts erzeugt aus dem optischen Kamerabild und dem Überlagerungsbild. Dabei wird automatisch eine stereoskopische Tiefenlage des optischen Kamerabildes auf die festgelegte stereoskopische Tiefenlage des Referenzpunktes festgelegt. In Abhängigkeit von dieser Tiefenlage wird eine stereoskopische Tiefenlage des Überlagerungsbildes in Bezug auf die stereoskopische Tiefenlage des Referenzpunktes so eingestellt, dass in der resultierenden stereoskopischen Darstellung das Überlagerungsbild zumindest in dem Referenzpunkt in Aufnahmerichtung des optischen Kamerabildes vor und/oder hinter dem optisch undurchsichtigen Teil erscheint, also eingeblendet oder dargestellt wird, gemäß einer entsprechenden realen Lage vor und/oder hinter dem optisch undurchsichtigen Teil. In der resultierenden stereoskopischen Darstellung kann das Überlagerungsbild also zumindest in dem Referenzpunkt jeweiligen realen Verhältnissen entsprechend in Aufnahmerichtung des optischen Kamerabildes vor und/oder hinter dem optisch undurchsichtigen Teil erscheinend dargestellt werden. Die realen räumlichen Verhältnisse geben dabei an, wie in der Realität der undurchsichtige Teil und die verdeckte Struktur räumlich zueinander angeordnet sind und/oder wie ein als Teil des Überlagerungsbildes dargestelltes Objekt in der Realität oder übertragen auf die Realität in Bezug auf, also relativ zu dem undurchsichtigen Teil und/oder zu der verdeckten Struktur angeordnet wäre. Es sei hier explizit auf die entsprechenden Erläuterungen im Zusammenhang mit dem erfindungsgemäßen bildgebenden System verwiesen, die entsprechend für das erfindungsgemäße Verfahren gelten.

Das erfindungsgemäße Verfahren kann also ein Betriebsverfahren für das erfindungsgemäße bildgebende System sein oder als solches aufgefasst werden. Es sei hier insbesondere hervorgehoben, dass ein chirurgischer Schritt, beispielsweise zum Positionieren der Kamera und/oder eines die Struktur bildenden oder zum Manipulieren der Struktur vorgesehenen Instruments oder Werkzeugs, nicht als Teil des erfindungsgemäßen Verfahrens beansprucht ist. Ein solcher chirurgischer Schritt ist also weder Teil des beanspruchten Verfahrens noch zur Durchführung oder Anwendung des beanspruchten Verfahrens notwendig. Gleichwohl kann das erfindungsgemäße Verfahren vorteilhaft während oder parallel zu einem interventionellen Eingriff angewendet werden. In einem medizinischen Anwendungsfall kann das erfindungsgemäße Verfahren ebenso beispielsweise auf außerhalb des menschlichen oder tierischen Körpers befindliche Gewebeproben oder Gewebeteile angewendet werden. Ebenso kann völlig ohne Eingriff das Kamerabild beispielsweise von außerhalb des Körpers aufgenommen werden, wobei der undurchsichtige Teil dann beispielsweise eine Hautoberfläche sein kann. Die optisch hierdurch verdeckte Struktur kann beispielsweise ein Knochen oder ein künstliches metallisches Objekt sein, welches mittels eines Röntgengerätes als materialdurchdringender Erfassungsmodalität erfasst oder abgebildet wird. Ebenso kann es beispielsweise möglich sein, das Kamerabild mittels einer ohne chirurgischen Eingriff positionierten verschluckbaren Kapsel-Kamera aufzunehmen.

Darüber hinaus kann das erfindungsgemäße Verfahren in anderen, außerhalb der Medizin oder Medizintechnik liegenden Anwendungsbereichen nutzbringend eingesetzt werden. So ist es beispielsweise möglich, Reparaturen oder Diagnoseuntersuchungen von Maschinen oder Geräten mittels des erfindungsgemäßen Verfahrens zu unterstützen, wobei sich der Vorteil ergibt, dass die jeweilige Maschine oder das jeweilige Gerät nicht zerlegt werden muss und dennoch eine präzise und verlässliche räumliche Orientierung und Navigation in einem jeweiligen Innenbereich ermöglicht wird.

In vorteilhafter Ausgestaltung der vorliegenden Erfindung wird als der Referenzpunkt ein Durchtrittspunkt eines Instruments oder Werkzeugs durch den optisch undurchsichtigen Teil bestimmt. Der Durchtrittspunkt ist also ein Punkt oder Bereich, in welchem das Instrument oder Werkzeug den undurchsichtigen Teil durchgreift und somit aus einem durch das Kamerabild abgebildeten Bereich in einen durch den undurchsichtigen Teil verdeckten Bereich übergeht. Dieser Durchtrittspunkt kann auch als Instrumenteneintrittspunkt bezeichnet werden. Wird als die materialdurchdringenden Bildgebungs- oder Erfassungsmodalität beispielsweise ein Röntgen- oder Fluoroskopiegerät verwendet, so ist dieser Durchtritts- oder Instrumenteneintrittspunkt in entsprechenden Röntgen- oder Fluoroskopiebildern beispielsweise als Übergangspunkt zwischen Luft und einem festen Gewebe oder Material entlang des Instruments erkennbar und identifizierbar. Die Verwendung dieses Durchtrittspunktes als der Referenzpunkt ist besonders vorteilhaft, da dieser üblicherweise besonders genau und eindeutig, also zuverlässig identifizierbar ist und zudem vorteilhaft stets ohnehin durch die Kamera abgebildet werden soll. Weiterhin ermöglicht die Wahl des Durchtrittspunktes als Referenzpunkt vorteilhaft eine besonders genaue und zuverlässige Darstellung, wenn beispielsweise das Instrument oder ein Modell des Instruments als Teil des Überlagerungsbildes erzeugt oder eingeblendet wird. Dies ist der Fall, da die Tiefenlage des Referenzpunktes eindeutig bestimmt und realitätsgetreu ist, sodass also auch dann wenn der undurchsichtige Teil seinerseits eine räumliche Tiefenausdehnung aufweist keinerlei optische Diskrepanzen auftreten und eine besonders präzise Anknüpfung des Überlagerungsbildes an das Kamerabild möglich ist.

In weiterer vorteilhafter Ausgestaltung der vorliegenden Erfindung wird als Teil des Überlagerungsbildes ein 3D-Modell der optisch verdeckten Struktur erzeugt. In weiterer vorteilhafter Ausgestaltung der vorliegenden Erfindung wird als Teil des Überlagerungsbildes ein Bewegungspfad für ein vorgegebenes Objekt erzeugt. Der Bewegungspfad kann beispielsweise ein geplanter Pfad sein, entlang welchem das Objekt geführt werden soll. Ebenso kann der Bewegungspfad ein erfasster und nachverfolgte Pfad, also eine bisherige Trajektorie, des vorgegebenen Objekts sein. Das vorgegebene Objekt kann beispielsweise ein Instrument oder ein Werkzeug sein. Ebenso kann das Objekt aber beispielsweise ein Fremdkörper oder ein Teil des Zielobjekts selbst sein, welcher beispielsweise entlang des Bewegungspfades in eine vorgegebene Zielposition verbracht werden soll. Die verdeckte Struktur kann beispielsweise Teil des Zielobjekts und/oder das Instrument oder Werkzeug sein. Dadurch, dass das 3D-Modell der Struktur als virtuelles Objekt erzeugt und als Teil des Überlagerungsbildes verwendet wird, kann dieses in der stereoskopischen Darstellung vorteilhaft besonders präzise und leicht erkennbar dargestellt werden. Somit kann also insgesamt eine verbesserte Erkennbarkeit der stereoskopischen Darstellung ebenso wie eine besonders präzise und einfache Orientierung und Navigation ermöglicht werden. Wie bereits erläutert kann sich der Bewegungspfad dabei insbesondere zumindest teilweise in die Tiefenrichtung erstrecken, was erst durch die mittels der vorliegenden Erfindung erzeugte stereoskopische Darstellung zuverlässig und in eindeutig erkennbarer und zu der Darstellung des Kamerabildes konsistenter Weise ermöglicht wird.

In weiterer vorteilhafter Ausgestaltung der vorliegenden Erfindung wird eine 2D-Position des Referenzpunktes in einer senkrecht zu der Aufnahmerichtung des Kamerabildes stehenden Bildebene des Kamerabildes bestimmt. Mit anderen Worten werden also X- und Y-Koordinaten des Referenzpunktes in dem 2D-Kamerabild bestimmt. Diese 2D-Position des Referenzpunktes in dem Kamerabild wird dann auch zur 2D-Registrierung des Überlagerungsbildes zu dem Kamerabild in der entsprechenden Bildebene verwendet. Mit anderen Worten wird also das Überlagerungsbild beziehungsweise die diesem zugrunde liegenden Erfassungsdaten in X- und Y-Richtung, also senkrecht zu der Tiefenrichtung verschoben, sodass sich in einem entsprechenden gemeinsamen Koordinatensystem des Kamerabildes und der Erfassungsdaten beziehungsweise des Überlagerungsbildes der Referenzpunkt an der gleichen 2D-Position befindet, also die gleichen X- und Y-Koordinaten aufweist. Ebenso kann analog selbstverständlich das Kamerabild in entsprechender Weise relativ zu dem Überlagerungsbild beziehungsweise den entsprechenden Erfassungsdaten verschoben werden. Durch die Verwendung des Referenzpunktes nicht nur als Tiefenreferenz, sondern auch als Referenz für die 2D-Position kann vorteilhaft besonders einfach und konsistent eine zuverlässige Registrierung und somit eine möglichst fehler- und artefaktfreie Darstellung der stereoskopischen Darstellung erreicht werden, da der Referenzpunkt ohnehin in beiden Datensätzen eindeutig identifizierbar ist beziehungsweise per Definition oder Anforderung sein muss.

In weiterer vorteilhafter Ausgestaltung der vorliegenden Erfindung wird ein optisch und mittels der materialdurchdringenden Erfassungsmodalität detektierbarer, also erfassbare oder abbildbarer, Marker zum Kennzeichnen des Referenzpunktes verwendet. Mit anderen Worten wird also der Marker, also ein entsprechendes Markierungsobjekt, an dem Referenzpunkt oder in einer fester Ortsbeziehung, also einer fester räumlichen Lagebeziehung, zu dem Marker angeordnet, insbesondere bevor das Überlagerungsbild erzeugt oder sogar bevor die entsprechenden Erfassungsdaten erfasst oder aufgenommen werden. Ebenso kann der Marker zunächst positioniert werden und dann die Position des Markers oder ein in einer vorgegebenen festen räumlichen Lagebeziehung zu der Position des Markers stehender Punkt als der Referenzpunkt verwendet werden. Hierdurch wird eine besonders zuverlässige und eindeutige Bestimmung der Lage oder Position des Referenzpunktes sowohl in dem Kamerabild als auch in den Erfassungsdaten der materialdurchdringenden Erfassungsmodalität ermöglicht. Der Marker kann beispielsweise eine Metallklammer oder ein an sich bekannter röntgensichtbarer beziehungsweise röntgenopaker Marker sein.

Die Verwendung eines solchen Markers kann beispielsweise dann besonders vorteilhaft sein, wenn der in dem Kamerabild abgebildete undurchsichtige Teil keine zuverlässig und eindeutig identifizierbaren Strukturen aufweist und/oder beispielsweise in sich beweglich oder flexibel ist. Der Marker kann beispielsweise dann besonders vorteilhaft verwendet werden, wenn das Instrument oder Werkzeug noch nicht durch den undurchsichtigen Teil hindurchgeführt ist, also noch kein entsprechender Durchtritts- oder Instrumenteneintrittspunkt existiert, oder überhaupt kein Instrument eingesetzt wird. Letzteres kann der Fall sein, da die vorliegende Erfindung rein für Abbildungszwecke verwendet werden kann.

In weiterer vorteilhafter Ausgestaltung der vorliegenden Erfindung wird mittels der materialdurchdringenden Erfassungsmodalität, also in den entsprechenden Erfassungsdaten, ein zum Aufnehmen des Kamerabildes verwendeter Teil der Erfassungseinrichtung, also beispielsweise die Kamera oder ein Kopf oder Endstück eines Endoskops, in dem die Kamera angeordnet ist, erfasst. Aus den entsprechenden Erfassungsdaten wird dann eine Lage und Blickrichtung dieses Teils der Erfassungseinrichtung, also insbesondere der Kamera, in Bezug auf den Referenzpunkt und/oder in Bezug auf den undurchsichtigen Teil des Zielobjekts bestimmt. Es kann also beispielsweise eine Fläche oder Ebene definiert werden, in der sich der Referenzpunkt befindet und die zumindest im Wesentlichen einer entsprechenden Oberfläche des undurchsichtigen Teils entspricht oder sich zumindest im Wesentlichen entlang dieser Oberfläche oder in deren Haupterstreckungsebene erstreckt, und dann ein Vektor zwischen der Kamera und dem Referenzpunkt beziehungsweise der definierten Ebene bestimmt werden. Dabei kann insbesondere ein Abstand zwischen der Kamera und dem Referenzpunkt beziehungsweise ein Abstand zwischen der Kamera und dem undurchsichtigen Teil des Zielobjekts bestimmt werden. Hierdurch wird vorteilhaft unter Berücksichtigung von bekannten inhärenten Abbildungseigenschaften der Kamera eine Größenbestimmung oder Festlegung einer Skalierung oder Skala für das Kamerabild und darin abgebildeter Strukturen ermöglicht. Um die Lage und Blickrichtung der Kamera in Bezug auf den Referenzpunkt beziehungsweise auf den undurchsichtigen Teil zuverlässig und genau bestimmen zu können, kann es insbesondere vorgesehen sein, dass eine Abbildungs- oder Erfassungsrichtung, in welcher die materialdurchdringenden Bildgebungs- oder Erfassungsmodalität die Struktur und die Kamera, also das Zielobjekt abbildet oder erfasst, nicht kollinear zur Aufnahmerichtung des Kamerabildes angeordnet ist. Die materialdurchdringenden Erfassungsmodalität kann also so angeordnet oder ausgerichtet werden, dass ein entsprechender Bereich seitlich, bevorzugt zumindest im Wesentlichen senkrecht, zu der Aufnahmerichtung der Kamera abgebildet oder erfasst wird.

Ebenso kann vorteilhaft die Tiefenlage des Referenzpunktes für die stereoskopische Darstellung dann so festgelegt werden, dass darin ein virtueller oder stereoskopischer Abstand zwischen der Bildschirm- oder Anzeigeebene und dem Referenzpunkt der tatsächlichen realen Entfernung zwischen der Kamera und dem als Referenzpunkt verwendeten Teil oder Bereich des undurchsichtigen Teils des Zielobjekts entspricht. Dadurch kann vorteilhaft eine besonders realistische Darstellung erreicht und zudem eine besonders genaue und bedarfsgerechte Positionierung der Kamera ermöglicht werden. Zudem wird eine Registrierung der Erfassungsdaten zu dem Kamerabild und eine konsistente Skalierung oder Größendarstellung des Überlagerungsbildes erleichtert. Dies ist nicht ohne weiteres trivial, da die materialdurchdringenden Erfassungsmodalität offensichtlich grundlegend andere Abbildungseigenschaften aufweisen kann als die zum Aufnehmen des Kamerabildes verwendete Kamera.

In weiterer vorteilhafter Ausgestaltung der vorliegenden Erfindung wird in der stereoskopischen Darstellung das Kamerabild in einem Bereich, in dem dieses mit dem Überlagerungsbild überlappt, automatisch angepasst durch Ausblenden (englisch: "Virtual Window"), Ausgrauen, Unscharfzeichnen und/oder teiltransparente Darstellung. Auf diese Weise kann vorteilhaft der Tiefeneindruck unterstützt, also verstärkt oder verbessert, und somit die stereoskopische Tiefenbeziehung zwischen dem Überlagerungsbild und den übrigen Bereichen des Kamerabildes leichter erkennbar gemacht werden. Dabei ist es besonders vorteilhaft möglich, dass das Überlagerungsbild über einen Rand oder ein Blickfeld, also einen Abbildungsbereich, des Kamerabildes hinausragt, die entsprechenden eingeblendeten Strukturen oder Überlagerungen also zumindest teilweise auch außerhalb des Kamerabildes dargestellt werden. Dazu kann ein Abbildungsbereich oder Blickfeld (englisch "Field of View") der stereoskopischen Darstellung also über das Blickfeld des Kamerabildes hinaus erweitert werden. Hierdurch kann dem Betrachter vorteilhaft ein größerer Kontext zur verbesserten Orientierung und Navigation geboten werden. Dies ist besonders vorteilhaft, da heutzutage verfügbare Endoskopkameras üblicherweise ein relativ kleines Blickfeld aufweisen.

In weiterer vorteilhafter Ausgestaltung der vorliegenden Erfindung wird das Kamerabild als 2D-Bild, also monoskopisch, aufgenommen und durch das Festlegen seiner stereoskopischen Tiefenlage auf die stereoskopische Tiefenlage des Referenzpunktes in der stereoskopischen Darstellung vollständig und einheitlich in dieser stereoskopischen Tiefenlage dargestellt wird. Besonders bevorzugt kann in dieser Ausführungsform der Erfindung zudem vorgesehen sein, dass als das Überlagerungsbild ein 3D-Modell oder eine 3D-Struktur dem 2D-Kamerabild überlagert, also mit diesem zum Erzeugen der stereoskopischen Darstellung kombiniert wird. Auf diese Weise kann vorteilhaft mit besonders geringem Aufwand bei dem Betrachter ein verbesserter Tiefeneindruck erzeugt und somit eine Orientierung oder Navigation erleichtert werden.

In weiterer vorteilhafter Ausgestaltung der vorliegenden Erfindung wird das Kamerabild als 2D-Bild, also monoskopisch, aufgenommen und vor dem Erzeugen der stereoskopischen Darstellung mittels eines 2D-zu-3D-Konversionsverfahrens konvertiert, also etwa in ein stereoskopisches Kamerabild umgewandelt. Hierdurch kann bereits eine zumindest angenäherte, unterstützende Tiefenwirkung erzielt werden. Die stereoskopische Darstellung wird dann erzeugt aus dem konvertierten Kamerabild und dem Überlagerungsbild, wobei zum Festlegen der stereoskopischen Tiefenlage des konvertierten Kamerabildes ein den Referenzpunkt umfassender Teilbereich des konvertierten Kamerabildes auf die stereoskopische Tiefenlage des Referenzpunktes festgelegt wird. Auf diese Weise kann vorteilhaft die definierte Tiefen-Lagebeziehung zwischen dem Referenzpunkt, dem undurchsichtigen Teil und dem Überlagerungsbild beibehalten oder aufrechterhalten und gleichzeitig eine realistischere Darstellung erreicht werden. Insbesondere kann dem Umstand Rechnung getragen werden, dass der undurchsichtige Teil des Zielobjekts in der Realität eine räumliche Ausdehnung in Tiefenrichtung aufweisen kann, welche von einem menschlichen Betrachter zumindest teilweise auch aus einem 2D-Kamerabild abgeleitet oder unbewusst rekonstruiert werden kann. Durch die Konvertierung des 2D-Kamerabildes wird also vorteilhaft eine natürliche Tiefenwahrnehmung des Betrachters unterstützt, wodurch eine weiter verbesserte Erkennbarkeit, Orientierung und räumliche Navigation erreicht werden kann, da mögliche Wahrnehmungskonflikte vermieden oder minimiert werden können.

In vorteilhafter Weiterbildung der vorliegenden Erfindung wird zunächst eine 3D-Aufnahme des optisch undurchsichtigen Teils aufgenommen und ein daraus, beispielsweise automatisch oder teilautomatisch erzeugtes 3D-Modell des optisch undurchsichtigen Teils dann, bevorzugt automatisch, zu dem 2D-Kamerabild registriert. Das 3D-Modell des undurchsichtigen Teils wird dann als Basis oder Grundlage beziehungsweise als Referenz für das Konvertieren des 2D-Kamerabildes verwendet. Die 3D-Aufnahme kann den mittels der materialdurchdringenden Erfassungsmodalität aufgenommenen Erfassungsdaten entsprechen, in oder mittels welchen auch die optisch verdeckte Struktur und gegebenenfalls die Kamera erfasst oder abgebildet werden. Ebenso kann die 3D-Aufnahme separat während des Verfahrens oder im Vorhinein aufgenommen werden. Die 3D-Aufnahme kann also beispielsweise bereits mehrere Tage oder gar Wochen vor dem Kamerabild aufgenommen und in der Zwischenzeit zum Erzeugen des 3D-Modells verarbeitet oder aufbereitet, beispielsweise segmentiert, werden. Die 3D-Aufnahme kann beispielsweise ein 3D-Röntgenbild, ein MRT-Datensatz oder dergleichen sein. Während sich herkömmliche Methoden zur 2D-zu-3D-Konvertierung beispielsweise auf Schattenwürfe, Konturverläufe, unterschiedliche Schärfegrade oder dergleichen verlassen müssen, wird durch die vorliegend vorgesehene 3D-Aufnahme und das daraus abgeleitete 3D-Modell eine realitätsgetreuere Konvertierung des 2D-Kamerabildes ermöglicht. Oftmals wird ohnehin eine solche 3D-Aufnahmen des entsprechenden Bereiches des Zielobjekts angefertigt, sodass die Verwendung der entsprechenden Daten in der beschriebenen Art und Weise besonders aufwands- und belastungsarm und effizient möglich ist.

Ein weiterer Aspekt der vorliegenden Erfindung ist ein Computerprogramm oder Computerprogrammprodukt, welches die Verfahrensschritte zumindest einer Ausführungsform des erfindungsgemäßen Verfahrens kodiert oder repräsentiert und zum Laden in einen, insbesondere elektronischen und/oder elektronisch lesbaren, Datenspeicher eines bildgebenden Systems zum Ausführen der Verfahrensschritte, also des Verfahrens, eingerichtet ist. Das erfindungsgemäße Computerprogramm kann also Programm-Mittel umfassen, um das erfindungsgemäße Verfahren auszuführen, wenn das erfindungsgemäße Computerprogramm durch das erfindungsgemäße bildgebende System ausgeführt wird. Dazu kann der Datenspeicher insbesondere mit einer Datenverarbeitungseinrichtung des bildgebenden Systems verbunden sein, welche zum Ausführen des erfindungsgemäßen Computerprogramms eingerichtet ist.

Ein weiterer Aspekt der vorliegenden Erfindung ist ein, insbesondere elektronischer und/oder elektronisch lesbarer, Datenspeicher oder Datenträger, in oder auf welchem ein erfindungsgemäßes Computerprogramm gespeichert ist. Der Datenspeicher kann insbesondere ein Datenspeicher für eine Datenverarbeitungseinrichtung und/oder ein Steuergerät des bildgebenden Systems sein. In dem erfindungsgemäßen Datenspeicher können, insbesondere als Teil des gespeicherten Programmcodes, zudem weitere Steueranweisungen für das bildgebende System, die Datenverarbeitungseinrichtung und/oder das Steuergerät gespeichert oder kodiert sein. Der auf dem erfindungsgemäßen Datenspeicher gespeicherten Programmcode ist also insbesondere dazu ausgestaltet und eingerichtet, bei einer Verwendung des Datenspeichers in dem bildgebenden System und bei einer Ausführung des Programmcodes durch das bildgebende System, durch die Datenverarbeitungseinrichtung oder durch das Steuergerät zumindest eine Ausführungsform des erfindungsgemäßen Verfahrens auszuführen oder deren Ausführung zu bewirken.

Das erfindungsgemäße bildgebende System kann insbesondere einen derartigen erfindungsgemäßen Datenspeicher umfassen. Das erfindungsgemäße bildgebende System, insbesondere dessen Datenverarbeitungseinrichtung, kann zudem eine Prozessoreinrichtung, beispielsweise einen Mikroprozessor und/oder Mikrocontroller, aufweisen, welche mit dem Datenspeicher verbundenen und zum Ausführen des auf diesem gespeicherten Programmcodes, also zum Ausführen des erfindungsgemäßen Computerprogramms eingerichtet ist.

Das im Zusammenhang mit dem erfindungsgemäßen Verfahren, dem erfindungsgemäßen Computerprogramm und/oder den erfindungsgemäßen Datenspeicher genannte bildgebende System kann also insbesondere das erfindungsgemäße bildgebende System sein und entsprechend umgekehrt. Das erfindungsgemäße bildgebende Gerät, das erfindungsgemäße Computerprogramm und der erfindungsgemäßen Datenspeicher können dementsprechend also einige oder alle der im Zusammenhang mit den übrigen Aspekten der vorliegenden Erfindung, insbesondere im Zusammenhang mit dem erfindungsgemäßen Verfahren, genannten Eigenschaften und/oder Komponenten aufweisen und umgekehrt.

Die bisher und im Folgenden angegebenen Eigenschaften und Weiterbildungen des erfindungsgemäßen bildgebenden Systems und des erfindungsgemäßen Verfahrens sowie die entsprechenden Vorteile sind jeweils sinngemäß wechselseitig zwischen diesen und ebenso auf die anderen Aspekte der Erfindung, also auf das erfindungsgemäße Computerprogramm und den erfindungsgemäßen Datenspeicher übertragbar und umgekehrt. Es gehören also zu der Erfindung auch solche Weiterbildungen des erfindungsgemäßen bildgebenden Geräts, des erfindungsgemäßen Verfahrens, des erfindungsgemäßen Computerprogramms und des erfindungsgemäßen Datenspeichers, welche Ausgestaltungen aufweisen, die hier zur Vermeidung unnötiger Redundanz nicht explizit in der jeweiligen Kombination oder für jeden Aspekt der Erfindung separat beschrieben sind.

Weitere Merkmale, Einzelheiten und Vorteile der vorliegenden Erfindung ergeben sich aus der nachfolgenden Beschreibung bevorzugter Ausführungsbeispiele sowie anhand der Zeichnungen. Dabei zeigen:
- FIG 1: einen beispielhaften schematischen Ablaufplan eines Verfahrens zum Erzeugen einer stereoskopischen Darstellung eines Zielobjekts;
- FIG 2: eine schematische Darstellung eines bildgebenden Systems zum Erzeugen einer stereoskopischen Darstellung eines Zielobjekts;
- FIG 3: eine erste schematische Übersichtsdarstellung zur Veranschaulichung des Verfahrens;
- FIG 4: eine zweite schematische Übersichtsdarstellung zur Veranschaulichung des Verfahrens.

Bei den im Folgenden erläuterten Ausführungsbeispielen handelt es sich um bevorzugte Ausführungsformen der Erfindung. Bei den Ausführungsbeispielen stellen die beschriebenen Komponenten der Ausführungsformen jeweils einzelne, unabhängig voneinander zu betrachtende Merkmale der Erfindung dar, welche die Erfindung jeweils auch unabhängig voneinander weiterbilden und damit auch einzeln oder in einer anderen als der gezeigten Kombination als Bestandteil der Erfindung anzusehen sind. Des Weiteren sind die beschriebenen Ausführungsformen auch durch weitere der bereits beschriebenen Merkmale der Erfindung ergänzbar.

In den Figuren sind gleiche, funktionsgleiche oder einander entsprechende Elemente jeweils mit den gleichen Bezugszeichen gekennzeichnet.

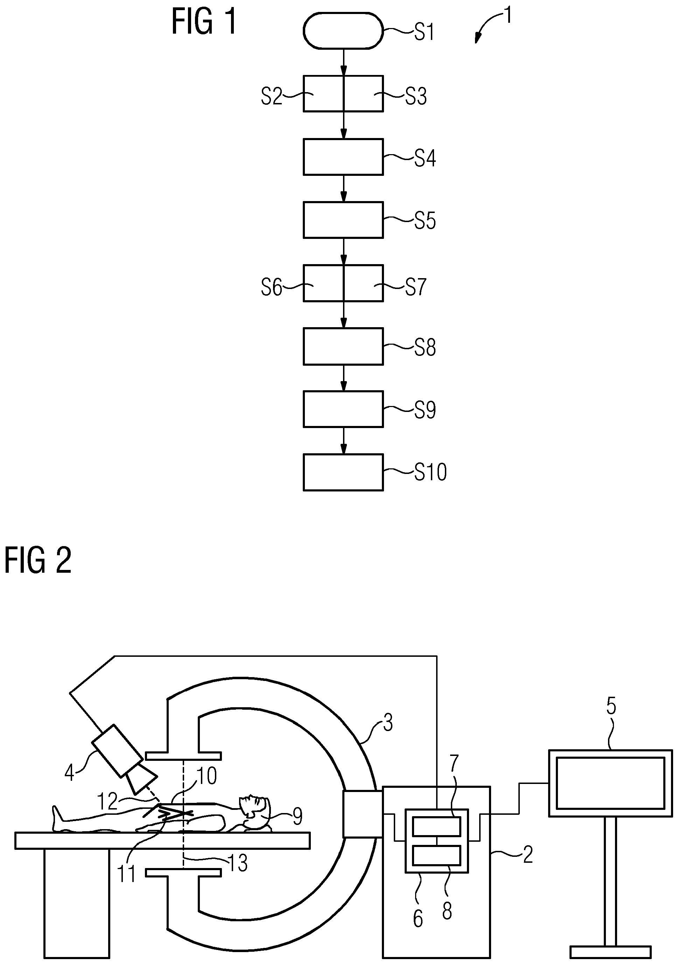

FIG 1 zeigt einen beispielhaften schematischen Ablaufplan 1 für ein Verfahren zum Erzeugen einer stereoskopischen Darstellung eines Zielobjekts. Dieses Verfahren wird weiter unten unter Bezugnahme auf die übrigen Figuren näher erläutert.
FIG 2 zeigt eine schematische Darstellung eines bildgebenden Systems 2 zum Erzeugen der stereoskopischen Darstellung des Zielobjekts nach dem genannten entsprechenden Verfahren.

Das bildgebende System 2 umfasst vorliegend als materialdurchdringende Bildgebungs- oder Erfassungsmodalität ein Röntgengerät 3. Zudem umfasst das bildgebende System 2 eine optische Kamera 4. Die hier dargestellten Ausführungsformen des Röntgengeräts 3 und der Kamera 4 sind rein beispielhaft und schematisch zu verstehen, sodass auch anders gestaltete entsprechende Geräte verwendet werden können. Weiter umfasst das bildgebende System 2 eine Anzeigeeinrichtung 5, bei der es sich vorliegend um einen 3D-Monitor handeln soll. Das Röntgengerät 3, die Kamera 4 und die Anzeigeeinrichtung 5 sind vorliegend mit einer Datenverarbeitungseinrichtung 6 des bildgebenden Systems 2 verbunden. Die Datenverarbeitungseinrichtung 6 umfasst ihrerseits eine Prozessoreinrichtung 7 zum Verarbeiten von mittels der Kamera 4 aufgenommenen Kamerabildern und mittels des Röntgengeräts 3 genommenen Erfassungsdaten sowie zum Ausgeben entsprechender Verarbeitungsergebnisse, hier also der zu erzeugenden stereoskopischen Darstellung, an die Anzeigeeinrichtung 5. Weiter umfasst die Datenverarbeitungseinrichtung 6 einen mit der Prozessoreinrichtung 7 verbundenen Datenspeicher 8, auf welchem ein Computerprogramm gespeichert ist, welches die Verfahrensschritte des genannten Verfahrens kodiert.

Das Verfahren wird in einem Verfahrensschritt S1 gestartet. Hier kann beispielsweise als das Zielobjekt ein Patient 9 in einem Aufnahmebereich des Röntgengeräts 3 und der Kamera 4 positioniert sowie das bildgebende System 2 aktiviert werden.

In einem Verfahrensschritt S2 wird mittels der Kamera 4 ein optisches Kamerabild eines optisch undurchsichtigen Bereiches 10 des Patienten 9 erfasst. Beispielsweise gleichzeitig dazu wird in einem Verfahrensschritt S3 mittels des Röntgengeräts 3 ein Röntgenbild einer verdeckten Struktur 11 des Patienten 9 aufgenommen. Die verdeckte Struktur 11 ist dabei aus oder in einer Aufnahmerichtung 12 der Kamera 4 durch den optisch undurchsichtigen Bereich 10 verdeckt. Der optisch undurchsichtige Bereich 10 ist jedoch für das Röntgengerät 3 zumindest teilweise transparent, sodass das Röntgenbild die optisch verdeckte Struktur 11 abbildet. Eine Erfassungseinrichtung 13 des Röntgengeräts 3 ist dabei nicht kollinear zu der Aufnahmerichtung 12, also in einem von 0° und 180° verschiedenen Winkel, zu der Aufnahmerichtung 12 der Kamera 4 ausgerichtet. Dadurch weist das Kamerabild also eine andere Perspektive als das Röntgenbild auf.

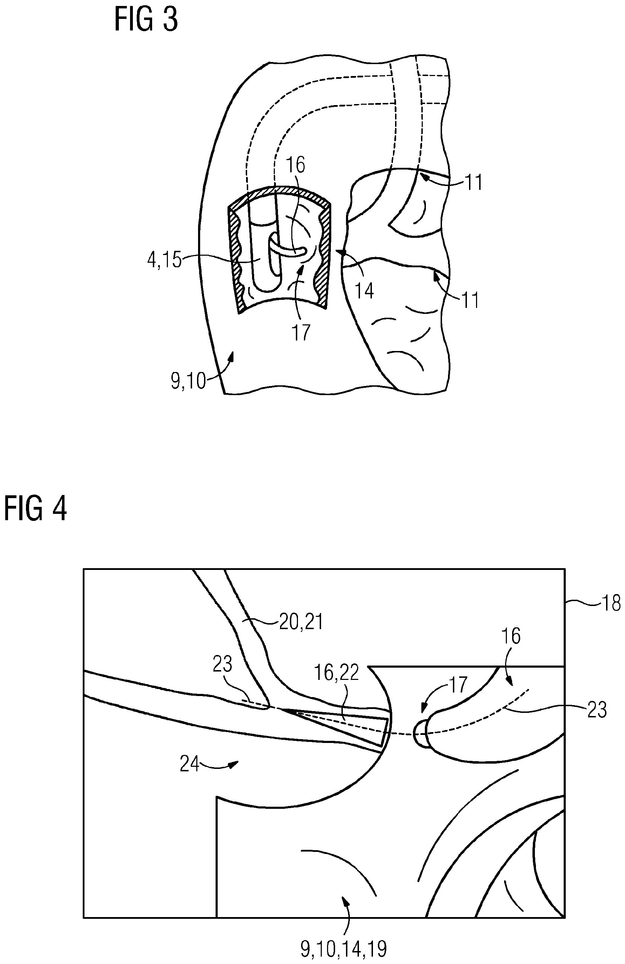

FIG 3 zeigt eine schematische Übersichtsdarstellung zur Veranschaulichung einer derartigen Situation. Hier ist der undurchsichtige Bereich 10 durch eine Gewebewand 14 eines Gefäßes des Patienten 9 gebildet. Als Kamera 4 ist hier ein Endoskop 15 in diesem Gefäß angeordnet. Eine Blick- oder Aufnahmerichtung des Endoskops 15 ist also auf die optisch undurchsichtige Gewebewand 14 gerichtet, sodass die räumlich dahinterliegende verdeckte Struktur 11 also durch das Endoskop 15 nicht abgebildet werden kann. Zusammen mit dem Endoskop 15 ist hier ein Instrument 16 in dem Gefäß angeordnet. Das Instrument 16 tritt dabei durch die Gewebewand 14 in Richtung der verdeckten Struktur 11 hindurch.

Beispielsweise bei einer Endoskopie eines Magen-Darmtrakts (Gastrointestinaltrakt), insbesondere bei der endoskopisch retrograden Cholangiopankreatikographie (ERCP) sehen mittels des flexiblen Endoskops 15 aufgenommene optische Kamerabilder oft relativ gleich aus. Das Endoskop 15 blickt auf eine Wand von Magen oder Darm und bildet nur das Instrument 16 bis zu einem Eintrittspunkt, an welchem das Instrument 16 die Wand, hier also die Gewebewand 14, durchtritt, ab. Das Instrument 16 kann beispielsweise eine Cannula, ein Führungsdraht, ein Katheter, eine Nadel oder dergleichen mehr sein. Das mittels des Endoskops 15 erfasste optische Kamerabild liefert keinerlei Informationen darüber, wo das Instrument 16 in einem aus Sicht des Endoskops 15 hinter der Gewebewand 14 liegenden Bereich in Relation zu dort befindlicher Anatomie, beispielsweise der verdeckten Struktur 11, angeordnet ist oder sich bewegt. Eine Orientierung in diesem Bereich hinter der Gewebewand 14 ist dann also üblicherweise nur durch einen separaten Blick auf ein beispielsweise senkrecht zur Blick- oder Aufnahmerichtung 12 des Endoskops 15 aufgenommenes Röntgen- oder Fluoroskopiebild möglich. Beispielsweise kann sich hinter der Gewebewand 14 ein geplanter Pfad erstrecken, entlang welchem das Instrument 16 zu der verdeckten Struktur 11 geführt werden soll. Es ist zu beachten, dass die Gewebewand 14, auf die das Endoskop 15 blickt, typischerweise nicht flach oder eben, sondern im Raum gewölbt ist, also eine Tiefenausdehnung in Richtung der Aufnahmerichtung 12 aufweist.

In einem Verfahrensschritt S4 werden durch die Datenverarbeitungseinrichtung 6 das mittels der Kamera 4 aufgenommene Kamerabild und das mittels des Röntgengeräts 3 aufgenommene Röntgenbild verarbeitet. Die Datenverarbeitungseinrichtung 6 bestimmt dabei einen in dem Kamerabild und in dem Röntgenbild abgebildeten, also identifizierbaren gemeinsamen Referenzpunkt 17. In dem in FIG 3 gezeigten Beispiel wird als der Referenzpunkt 17 ein Durchtrittspunkt bestimmt, in welchem das Instrument 16 durch die Gewebewand 14 hindurchtritt. Bei einer ERCP-Prozedur kann der Referenzpunkt 17 ein Eintrittspunkt einer Cannula oder eines anderen Instruments 16 in die Papille als Eintrittspunkt in den Gallentrakt sein. Bei laparoskopischen Ablationen kann der Referenzpunkt 17 ein Eintrittspunkt einer Ablationsnadel in eine entsprechende Organoberfläche sein. Ist bei der jeweiligen Anwendung kein solcher Durchtrittspunkt handeln, da beispielsweise kein Instrument 16 verwendet wird, kann ebenso ein anderer Punkt des Kamerabildes als der Referenzpunkt 17 verwendet werden, beispielsweise eine bestimmte markante Struktur des undurchsichtigen Teils 10. Es werden hier also 2D-Koordinaten des Referenzpunktes 17 in dem Kamerabild bestimmt und entsprechende 3D-Koordinaten des Referenzpunktes 17 anhand des Röntgen- oder Fluoroskopie Bildes bestimmt. Dazu können ebenso mehrere aus unterschiedlichen Erfassungsrichtungen 13 aufgenommene Röntgen- oder Fluoroskopiebilder verwendet werden.

In einem Verfahrensschritt S5 wird eine stereoskopische Tiefenlage des Referenzpunktes 17 auf einen vorgegebenen Wert festgelegt. Hier kann für den Referenzpunkt 17 im Allgemeinen eine Vergenz von 0 festgelegt werden, sodass in einer entsprechenden stereoskopischen Darstellung der Referenzpunkt 17 auf der Bildschirmoberfläche liegend erscheint oder eingeblendet würde. Ist jedoch die Kamera 4 in dem Röntgenbild mit abgebildet, so kann deren Abstand zu dem Referenzpunkt 17 beziehungsweise zu der Gewebewand 14, in welcher der Referenzpunkt 17 liegt, automatisch bestimmt und als Basis für die Festlegung der stereoskopischen Tiefenlage des Referenzpunktes 17 verwendet werden.

In einem optionalen Verfahrensschritt S6 wird das zweidimensionale Kamerabild mittels eines entsprechenden 2D-zu-3D-Konversionsverfahren in ein stereoskopisches Kamerabild umgewandelt. Alternativ wird das Kamerabild als 2D-Bild aufgenommen und als solches belassen. Beispielsweise gleichzeitig dazu wird in einem Verfahrensschritt S7 anhand des Röntgenbildes und der darin abgebildeten optisch verdeckten Struktur 11 ein Überlagerungsbild erzeugt. Dazu werden vorliegend die verdeckte Struktur 11 und ein aus Sicht des Endoskops 15 beziehungsweise der Kamera 4 hinter dem undurchsichtigen Teil 10 liegender Anteil des Instruments 16 virtuell dreidimensional modelliert. Zudem wird ein räumlicher 3D-Bewegungspfad 23 (siehe FIG 4) modelliert.

In einem Verfahrensschritt S8 werden das Kamerabild und das Überlagerungsbild, also die erzeugten virtuellen 3D-Modell, miteinander registriert, also in einem gemeinsamen Koordinatensystem entsprechend einer räumlichen Anordnung ihrer realen Gegenstücke virtuell angeordnet, insbesondere mit zumindest in dem Referenzpunkt 17 entsprechend konsistenten Tiefenlagen. Dabei wird eine stereoskopische Tiefenlage des Kamerabildes - zumindest in dem Referenzpunkt 17 - auf die stereoskopische Tiefenlage des Referenzpunktes 17 festgelegt. Ebenso wird eine stereoskopische Tiefenlage des Überlagerungsbildes auf die festgelegte stereoskopische Tiefenlage des Referenzpunktes 17 bezogen, also in Abhängigkeit von dieser festgelegt oder eingestellt. Es werden also die virtuellen 3D-Modelle des Überlagerungsbildes in dem gemeinsamen Koordinatensystem so verschoben, dass der Referenzpunkt 17 in dem Überlagerungsbild die gleiche Tiefenlage, also beispielsweise die gleiche Vergenz oder Parallaxe, aufweist wie in dem Kamerabild. Da hier also die stereoskopische Tiefenlage des Referenzpunktes 17 als Referenz zum Kombinieren des Kamerabildes und des Überlagerungsbildes verwendet wird, können die stereoskopischen Tiefenlagen in anderen Punkten oder Bereichen inkonsistent sein oder nicht notwendigerweise jeweiligen realen Verhältnissen entsprechen. Dies wird hier jedoch in Kauf genommen, da der Referenzpunkt im Extremfall der einzige zuverlässig bestimmte Berührungs- oder Überlappungspunkt zwischen in dem Kamerabild dargestellten Inhalten oder Objekten und darin nicht sichtbaren, also etwa durch die Gewebewand 14 verdeckten, Inhalten oder Objekten sein kann.

Weiter wird bei Bedarf eine Verschiebung in senkrecht zu der stereoskopischen Tiefenrichtung verlaufender seitlicher Richtung vorgenommen, sodass der Referenzpunkt 17 in dem Überlagerungsbild auch die gleichen 2D-Koordinaten wie in dem Kamerabild beziehungsweise wie der in dem Kamerabild abgebildete Referenzpunkt 17 in dem gemeinsamen 3D-Koordinatensystem hat.

In einem Verfahrensschritt S9 wird mittels der Datenverarbeitungseinrichtung 6 aus dem Kamerabild und dem Überlagerungsbild entsprechend, also unter Berücksichtigung, der im Verfahrensschritt S8 vorgenommenen Registrierung und virtuellen Positionierung die stereoskopische Darstellung, also ein zusammengesetztes Stereobild erzeugt. Sofern auf die 2D-zu-3D-Konversion im Verfahrensschritt S6 verzichtet wurde, kann also insbesondere das 2D-Kamerabild über seine gesamte Fläche mit der gleichen stereoskopischen Tiefenlage in der stereoskopischen Darstellung dargestellt werden, wobei darin dem 2D-Kamerabild 2D- oder 3D-Strukturen des Überlagerungsbildes überlagert dargestellt werden.

FIG 4 zeigt schematisch ein stereoskopisches Teilbild 18, also eine von zwei zum Erzeugen des zusammengesetzten Stereobildes verwendeten 2D-Ansichten. Hier ist als das Kamerabild ein mittels des Endoskops 15 aufgenommenes Endoskopbild 19 in einem Teilbereich des stereoskopischen Teilbildes 18 dargestellt. Als das Überlagerungsbild ist eine Einblendung 20 dargestellt. Die Einblendung 20 umfasst hier eine Modellstruktur 21, welche die verdeckte Struktur 11 repräsentiert, ein Instrumentenmodell 22, welches einen aus Sicht des Endoskops 15 hinter der Gewebewand 14 liegenden Anteil des Instruments 16 repräsentiert, und einen Bewegungspfad 23 des Instruments 16 beziehungsweise für das Instrument 16. Dieser Bewegungspfad 23 erstreckt sich dabei in der stereoskopischen Darstellung in Tiefenrichtung sowohl vor als auch hinter der Gewebewand 14. Die Modellstruktur 21 und das Instrumentenmodell 22 sind hier in einem virtuellen Fenster ("Virtual Window") und über einen Bildbereich des Endoskopbilds 19 hinausragend dargestellt.

Dazu ist das Endoskopbild 19 in einem Überlappungsbereich 24 zwischen der Einblendung 20 beziehungsweise der Modellstruktur 21 und dem Instrumentenmodell 22 einerseits und dem Endoskopbild 19 andererseits ausgeblendet. Meist hat der optische Blick des Endoskops 15 ein recht kleines Blickfeld. Das Instrument 16 und interessante anatomische Strukturen, wie beispielsweise die verdeckte Struktur 11 und dementsprechend hier die Modellstruktur 21, können sich dann zumindest teilweise außerhalb dieses Blickfeldes oder Aufnahmefeldes befinden. Deshalb ist hier das Blickfeld für das stereoskopische Teilbild 18 und somit für die stereoskopische Darstellung derart erweitert, dass alle relevanten Strukturen sichtbar sind. Das optische Endoskopbild 19 ist in 2D und 3D entsprechend nur in einem Ausschnitt oder Teilbereich des stereoskopischen Teilbildes 18 und dem entsprechend auch der stereoskopischen Darstellung dargestellt. Da die stereoskopische Tiefenlage des Endoskopbild 19 auf die feste stereoskopische Tiefenlage des Referenzpunktes 17 festgelegt ist, verbleibt das Endoskopbild 19 hier - zumindest sofern im Verfahrensschritt S6 auf die optionale 2D-zu-3D-Konversion verzichtet wurde - also in einer 2D-Ebene der stereoskopischen Darstellung mit fester Vergenz, während die Einblendung 20 stereoskopisch dargestellt wird und sich über einen ausgedehnten Tiefenbereich, also unterschiedliche Vergenzen oder Vergenzwerte erstreckt oder erstrecken kann.

In einem Verfahrensschritt S10 gibt die Datenverarbeitungseinrichtung 6 das erzeugte zusammengesetzte Stereobild, also die stereoskopische Darstellung an die Anzeigeeinrichtung 5 zur Darstellung aus.

Insgesamt zeigen die beschriebenen Beispiele wie eine verbesserte Abbildung eines zumindest teilweise optisch undurchsichtigen Zielobjekts ermöglicht werden kann. Dabei werden anatomische Strukturen, Geräte, Planungspfade und/oder dergleichen mehr, welche sich hinter dem undurchsichtigen Bereich befinden, in ein entsprechendes Kamerabild eingeblendet. Obwohl das Kamerabild dabei lediglich eine monoskopische Bildgebung aufweist, wird sichergestellt, dass in dem resultierenden zusammengesetzten Stereobild beziehungsweise der resultierenden stereoskopischen Darstellung dennoch eine sinnvolle, insbesondere realistische oder der Realität entsprechende, Tiefenbeziehung zwischen dem Kamerabild und den Einblendungen oder Überlagerungen besteht. Als Tiefenreferenz dient dabei ein bestimmter Punkt, auf den oder auf dessen stereoskopische Tiefenlage sämtliche anderen Einblendungen oder Teile der stereoskopischen Darstellung bezogen werden. Es kann hier also ausgehend von dem Bezugs- oder Referenzpunkt 17 eine geeignete Darstellung erzeugt werden, die aus einer Kamera- oder Endoskopsicht und einer virtuellen Überlagerung zusammengesetzt ist.

## Patentansprüche

1. Bildgebendes System (2) zum Erzeugen einer stereoskopischen Darstellung eines Zielobjekts (9), mit einer Erfassungseinrichtung (3, 4, 15) und einer Datenverarbeitungseinrichtung (6), wobei
- die Erfassungseinrichtung (3, 4, 15) dazu eingerichtet ist,
- ein optisches Kamerabild (19) eines optisch undurchsichtigen Teils (10, 14) des Zielobjekts (9) zu erfassen, und
- mittels einer materialdurchdringenden Erfassungsmodalität (3) eine in einer Aufnahmerichtung (12) des Kamerabildes (19) durch den undurchsichtigen Teil (10, 14) optisch verdeckte Struktur (11) zu erfassen, und
- die Datenverarbeitungseinrichtung (6) dazu eingerichtet ist, automatisch
- einen in dem Kamerabild (19) und mittels der materialdurchdringenden Erfassungsmodalität (3) abgebildeten gemeinsamen Referenzpunkt (17) zu bestimmen,
- eine stereoskopische Tiefenlage des Referenzpunktes (17) auf einen vorgegebenen Wert festzulegen,
- anhand der erfassten verdeckten Struktur (11) ein Überlagerungsbild (20) zu erzeugen, und
- die stereoskopische Darstellung aus dem Kamerabild (19) und dem Überlagerungsbild (20) zu erzeugen, und dabei automatisch eine stereoskopische Tiefenlage des Kamerabildes (19) auf die stereoskopische Tiefenlage des Referenzpunktes (17) festzulegen und in Abhängigkeit von dieser eine stereoskopische Tiefenlage des Überlagerungsbildes (20) in Bezug auf die stereoskopische Tiefenlage des Referenzpunktes (17) einzustellen, sodass in der stereoskopische Darstellung das Überlagerungsbild (20) zumindest in dem Referenzpunkt (17) in Aufnahmerichtung (12) des Kamerabildes (19) vor und/oder hinter dem optisch undurchsichtigen Teil (10, 14) erscheint gemäß einer entsprechenden realen Lage vor und/oder hinter dem optisch undurchsichtigen Teil (10, 14).

2. Verfahren (1) zum Erzeugen einer stereoskopischen Darstellung eines Zielobjekts mittels eines bildgebenden Systems (2), mit den Verfahrensschritten
- Erfassen eines optisches Kamerabildes (19) eines optisch undurchsichtigen Teils (10, 14) des Zielobjekts (9),
- Erfassen einer in einer Aufnahmerichtung (12) des optischen Kamerabildes (19) durch den undurchsichtigen Teil (10, 14) optisch verdeckten Struktur (11) mittels einer materialdurchdringenden Erfassungsmodalität (3),
- automatisches Bestimmen eines in dem Kamerabild (19) und mittels der materialdurchdringenden Erfassungsmodalität (3) abgebildeten gemeinsamen Referenzpunktes (17),
- automatisches Festlegen einer stereoskopischen Tiefenlage des Referenzpunktes (17) auf einen vorgegebenen Wert,
- automatisches Erzeugen eines Überlagerungsbildes (20) anhand der erfassten verdeckten Struktur (11),
- automatisches Erzeugen der stereoskopischen Darstellung aus dem optischen Kamerabild (19) und dem Überlagerungsbild (20), wobei automatisch eine stereoskopische Tiefenlage des optischen Kamerabildes (19) auf die stereoskopische Tiefenlage des Referenzpunktes (17) festgelegt wird und in Abhängigkeit von dieser eine stereoskopische Tiefenlage des Überlagerungsbildes (20) in Bezug auf die stereoskopische Tiefenlage des Referenzpunktes (17) eingestellt wird, sodass in der stereoskopische Darstellung das Überlagerungsbild (20) zumindest in dem Referenzpunkt (17) in Aufnahmerichtung (12) des optischen Kamerabildes (19) vor und/oder hinter dem optisch undurchsichtigen Teil (10, 14) erscheint gemäß einer entsprechenden realen Lage vor und/oder hinter dem optisch undurchsichtigen Teil (10, 14).

3. Verfahren (1) nach Anspruch 2, **dadurch gekennzeichnet, dass** als der Referenzpunkt (17) ein Durchtrittspunkt (17) eines Instruments (16) durch den optisch undurchsichtigen Teil (10, 14) bestimmt wird.

4. Verfahren (1) nach einem der Ansprüche 2 und 3, **dadurch gekennzeichnet, dass** als Teil des Überlagerungsbildes (20) ein 3D-Modell (21, 22) der optisch verdeckten Struktur (11) erzeugt wird.

5. Verfahren nach einem der Ansprüche 2 bis 4, **dadurch gekennzeichnet, dass** als Teil des Überlagerungsbildes (20) ein Bewegungspfad (23) für ein vorgegebenes Objekt (16) erzeugt wird.

6. Verfahren (1) nach einem der Ansprüche 2 bis 5, **dadurch gekennzeichnet, dass** eine 2D-Position des Referenzpunktes (17) in einer senkrecht zu der Aufnahmerichtung (12) des Kamerabildes (19) stehenden Bildebene des Kamerabildes (19) bestimmt und diese 2D-Position des Referenzpunktes (17) auch zur 2D-Registrierung des Überlagerungsbildes (20) zu dem Kamerabild (19) in der Bildebene verwendet wird.

7. Verfahren (1) nach einem der Ansprüche 2 bis 6, **dadurch gekennzeichnet, dass** ein optisch und mittels der materialdurchdringenden Erfassungsmodalität (3) detektierbarer Marker zum Kennzeichnen des Referenzpunktes (17) verwendet wird.

8. Verfahren (1) nach einem der Ansprüche 2 bis 7, **dadurch gekennzeichnet, dass** mittels der materialdurchdringenden Erfassungsmodalität (3) ein zum Aufnehmen des Kamerabildes (19) verwendeter Teil (4, 15) der Erfassungseinrichtung (3, 4, 15) erfasst wird, aus entsprechenden Erfassungsdaten eine Lage und Blickrichtung (12) dieses Teils (4, 15) der Erfassungseinrichtung (3, 4, 15) in Bezug auf den Referenzpunkt (17) bestimmt wird.

9. Verfahren (1) nach einem der Ansprüche 2 bis 8, **dadurch gekennzeichnet, dass** in der stereoskopischen Darstellung das Kamerabild (19) in einem Bereich (24), in dem dieses mit dem Überlagerungsbild (20) überlappt, automatisch angepasst wird, durch Ausblenden, Ausgrauen, Unscharfzeichnen und/oder teiltransparente Darstellung.

10. Verfahren (1) nach einem der Ansprüche 2 bis 9, **dadurch gekennzeichnet, dass** das Kamerabild (19) als 2D-Bild aufgenommen wird und durch das Festlegen seiner stereoskopischen Tiefenlage auf die stereoskopische Tiefenlage des Referenzpunktes (17) in der stereoskopischen Darstellung vollständig und einheitlich in dieser stereoskopischen Tiefenlage dargestellt wird.

11. Verfahren (1) nach einem der Ansprüche 2 bis 9, **dadurch gekennzeichnet, dass** das Kamerabild (19) als 2D-Bild aufgenommen wird und vor dem Erzeugen der stereoskopischen Darstellung mittels eines 2D-zu-3D-Konversionsverfahrens konvertiert wird, und die stereoskopische Darstellung dann erzeugt wird aus dem konvertierten Kamerabild und dem Überlagerungsbild (20), wobei zum Festlegen der stereoskopischen Tiefenlage des konvertierten Kamerabildes ein den Referenzpunkt (17) umfassender Teilbereich des konvertierten Kamerabildes auf die stereoskopische Tiefenlage des Referenzpunktes (17) festgelegt wird.

12. Verfahren (1) nach Anspruch 11, **dadurch gekennzeichnet, dass** zunächst eine 3D-Aufnahme des optisch undurchsichtigen Teils (10, 14) aufgenommen wird und ein daraus erzeugtes 3D-Modell des optisch undurchsichtigen Teils zu dem 2D-Kamerabild registriert und als Basis für das Konvertieren des 2D-Kamerabildes verwendet wird.

13. Computerprogramm, welches die Verfahrensschritte eines Verfahrens (1) nach einem der Ansprüche 2 bis 12 kodiert und zum Laden in einen Datenspeicher (8) eines bildgebenden Systems (2) zum Ausführen der Verfahrensschritte eingerichtet ist.

14. Datenspeicher (8) für ein bildgebendes System (2), wobei in dem Datenspeicher (8) ein Programmcode gespeichert ist, welcher zumindest ein Computerprogramm nach Anspruch 13 umfasst.

## Claims

1. Imaging system (2) for generating a stereoscopic representation of a target object (9), having an acquisition device (3, 4, 15) and a data processing device (6), wherein
- the acquisition device (3, 4, 15) is configured
- to acquire an optical camera image (19) of an optically opaque part (10, 14) of the target object (9), and
- to acquire, by means of a material-penetrating acquisition modality (3), a structure (11) which is optically concealed by the opaque part (10, 14) in a recording direction (12) of the camera image (19), and
- the data processing device (6) is configured
- to automatically specify a common reference point (17) which is depicted in the camera image (19) and by the material-penetrating acquisition modality (3),
- to automatically fix a stereoscopic depth location of the reference point (17) at a predetermined value,
- to automatically generate an overlay image (20) on the basis of the acquired concealed structure (11), and
- to automatically generate the stereoscopic representation from the camera image (19) and the overlay image (20), and in this case to automatically fix a stereoscopic depth location of the camera image (19) at the stereoscopic depth location of the reference point (17) and, as a function thereof, to adjust a stereoscopic depth location of the overlay image (20) in relation to the stereoscopic depth location of the reference point (17), such that in the stereoscopic representation the overlay image (20), at least at the reference point (17), appears in front of and/or behind the optically opaque part (10, 14) in the recording direction (12) of the camera image (19) in accordance with a corresponding real location in front of and/or behind the optically opaque part (10, 14).

2. Method (1) for generating a stereoscopic representation of a target object by means of an imaging system (2), having the following method steps:
- acquiring an optical camera image (19) of an optically opaque part (10, 14) of the target object (9),
- acquiring by means of a material-penetrating acquisition modality (3) a structure (11) which is optically concealed by the opaque part (10, 14) in a recording direction (12) of the optical camera image (19),
- automatically specifying a common reference point (17) which is depicted in the camera image (19) and by the material-penetrating acquisition modality (3),
- automatically fixing a stereoscopic depth location of the reference point (17) at a predetermined value,
- automatically generating an overlay image (20) on the basis of the acquired concealed structure (11),
- automatically generating the stereoscopic representation from the optical camera image (19) and the overlay image (20), wherein a stereoscopic depth location of the optical camera image (19) is automatically fixed at the stereoscopic depth location of the reference point (17) and, as a function thereof, a stereoscopic depth location of the overlay image (20) is adjusted in relation to the stereoscopic depth location of the reference point (17), such that in the stereoscopic representation the overlay image (20), at least at the reference point (17), appears in front of and/or behind the optically opaque part (10, 14) in the recording direction (12) of the optical camera image (19) in accordance with a corresponding real location in front of and/or behind the optically opaque part (10, 14).

3. Method (1) according to claim 2, **characterised in that** a penetration point (17) of an instrument (16) through the optically opaque part (10, 14) is specified as the reference point (17).

4. Method (1) according to one of claims 2 and 3, **characterised in that** a 3D model (21, 22) of the optically concealed structure (11) is generated as part of the overlay image (20).

5. Method according to one of claims 2 to 4, **characterised in that** a travel path (23) for a predetermined object (16) is generated as part of the overlay image (20).

6. Method (1) according to one of claims 2 to 5, **characterised in that** a 2D position of the reference point (17) is specified in an image plane of the camera image (19) which is perpendicular to the recording direction (12) of the camera image (19), and this 2D position of the reference point (17) is also used for the 2D registration of the overlay image (20) with the camera image (19) in the image plane.

7. Method (1) according to one of claims 2 to 6, **characterised in that** a marker which can be detected optically and by means of the material-penetrating acquisition modality (3) is used to indicate the reference point (17).

8. Method (1) according to one of claims 2 to 7, **characterised in that** a part (4, 15) of the acquisition device (3, 4, 15) which is used for recording the camera image (19) is acquired by means of the material-penetrating acquisition modality (3), and a location and viewing direction (12) of this part (4, 15) of the acquisition device (3, 4, 15) in relation to the reference point (17) is specified from corresponding acquisition data.

9. Method (1) according to one of claims 2 to 8, **characterised in that** in the stereoscopic representation, the camera image (19) is automatically adapted in a region (24) in which it overlaps with the overlay image (20), by means of masking, dimming, blurring and/or partially transparent representation.

10. Method (1) according to one of claims 2 to 9, **characterised in that** the camera image (19) is recorded as a 2D image and, by means of fixing its stereoscopic depth location at the stereoscopic depth location of the reference point (17) in the stereoscopic representation, is represented entirely and in an integrated manner at this stereoscopic depth location.

11. Method (1) according to one of claims 2 to 9, **characterised in that** the camera image (19) is recorded as a 2D image and is converted by means of a 2D to 3D conversion method before the stereoscopic representation is generated, and the stereoscopic representation is then generated from the converted camera image and the overlay image (20), wherein for the purpose of fixing the stereoscopic depth location of the converted camera image, a partial region of the converted camera image containing the reference point (17) is fixed at the stereoscopic depth location of the reference point (17).

12. Method (1) according to claim 11, **characterised in that** a 3D recording of the optically opaque part (10, 14) is recorded first, and a 3D model which is generated therefrom of the optically opaque part is registered with the 2D camera image and is used as a basis for the conversion of the 2D camera image.

13. Computer program which encodes the method steps of a method (1) according to one of claims 2 to 12 and is configured to be loaded into a data store (8) of an imaging system (2) in order to execute the method steps.

14. Data store (8) for an imaging system (2), wherein program code comprising at least one computer program according to claim 13 is stored in the data store (8).

## Revendications

1. Système (2) d'imagerie pour la production d'une représentation stéréoscopique d'un objet (9) cible, comprenant un dispositif (3, 4, 15) de détection et un dispositif (6) de traitement de données, dans lequel
- le dispositif (3, 4, 15) de détection est conçu pour :
- détecter une image (19) optique d'appareil photographique d'une partie (10, 14) opaque optiquement de l'objet (9) cible, et
- au moyen d'une modalité (3) de détection pénétrant dans la matière, détecter une structure (11) cachée optiquement par la partie (10, 14) opaque dans une direction (12) d'enregistrement de l'image (19) d'appareil photographique, et
- le dispositif (6) de traitement de données est conçu pour, automatiquement,
- déterminer un point (17) de référence commun reproduit dans l'image (19) d'appareil photographique et au moyen de la modalité (3) de détection pénétrant le matériau,
- fixer une position en profondeur stéréoscopique du point (17) de référence à une valeur donnée à l'avance,
- produire, à l'aide de la structure (11) cachée et détectée, une image (20) de superposition, et
- produire la représentation stéréoscopique à partir de l'image (19) d'appareil photographique et de l'image (20) de superposition et fixer ainsi automatiquement une position en profondeur stéréoscopique de l'image (19) d'appareil photographique à la position en profondeur stéréoscopique du point (17) de référence et, en fonction de celle-ci, régler une position en profondeur stéréoscopique de l'image (20) de superposition par rapport à la position en profondeur stéréoscopique du point (17) de référence, de manière à ce que, dans la représentation stéréoscopique, l'image (20) de superposition apparaisse au moins au point (17) de référence dans la direction (12) d'enregistrement de l'image (19) d'appareil photographique devant et/ou derrière la partie (10, 14) opaque optiquement suivant une position réelle correspondante devant et/ou derrière la partie (10, 14) opaque optiquement.

2. Procédé (1) de production d'une représentation stéréoscopique d'un objet cible au moyen d'un système (2) d'imagerie, comprenant les stades de procédé
- détection d'une image (19) optique d'appareil photographique d'une partie (10, 14) opaque optiquement de l'élément (9) cible,
- détection d'une structure (11) cachée optiquement par la partie (10, 14) opaque dans une direction (12) d'enregistrement de l'image (19) optique d'appareil photographique au moyen d'une modalité (3) de détection pénétrant dans le matériau,
- détermination automatique d'un point (17) de référence commun et reproduit dans l'image (19) d'appareil photographique et au moyen de la modalité (3) de détection pénétrant dans le matériau,
- fixation automatique d'une position en profondeur stéréoscopique du point (17) de référence à une valeur donnée à l'avance,
- production automatique d'une image (20) de superposition à l'aide de la structure (11) cachée détectée,
- production automatique de la représentation stéréoscopique à partir de l'image (19) optique d'appareil photographique et de l'image (20) de superposition, dans lequel on fixe automatiquement une position en profondeur stéréoscopique de l'image (19) optique de l'appareil photographique à la position en profondeur stéréoscopique du point (17) de référence et, en fonction de cela, on règle une position en profondeur stéréoscopique de l'image (20) de superposition par rapport à la position en profondeur stéréoscopique du point (17) de référence, de manière à ce que, dans la représentation stéréoscopique, une image (20) de superposition apparaisse au moins au point (17) de référence, dans la direction (12) d'enregistrement de l'image (19) d'appareil photographique devant et/ou derrière la partie (10, 14) opaque optiquement suivant une position réelle correspondante devant et/ou derrière la partie (10, 14) opaque optiquement.

3. Procédé (1) suivant la revendication 2, **caractérisé en ce que** l'on détermine comme point (17) de référence un point (17) de passage d'un instrument (16) dans la partie (10, 14) opaque optiquement.

4. Procédé (1) suivant l'une des revendications 2 et 3, **caractérisé en ce que** l'on produit comme partie de l'image (20) de superposition un modèle (21, 22) en 3D de la structure (11) cachée optiquement.

5. Procédé suivant l'une des revendications 2 à 4, **caractérisé en ce que** l'on produit comme partie de l'image (20) de superposition un trajet (23) de déplacement d'un objet (16) donné à l'avance.

6. Procédé (1) suivant l'une des revendications 2 à 5, **caractérisé en ce que** l'on utilise une position en 2D du point (17) de référence dans un plan d'image, perpendiculaire à la direction (12) d'enregistrement de l'image (19) d'appareil photographique, de l'image (19) d'appareil photographique et on utilise cette position en 2D du point (17) de référence également pour la mise en coïncidence en 2D de l'image (20) de superposition avec l'image (19) d'appareil photographique dans le plan d'image.

7. Procédé (1) suivant l'une des revendications 2 à 6, **caractérisé en ce que** l'on utilise pour caractériser le point (17) de référence un marqueur pouvant être détecté optiquement et au moyen de la modalité (3) de détection pénétrant le matériau.

8. Procédé (1) suivant l'une des revendications 2 à 7, **caractérisé en ce qu'**au moyen de la modalité (3) de détection traversant le matériau, on détecte une partie (4, 15) du dispositif (3, 4, 15) de détection utilisée pour l'enregistrement de l'image (19) d'appareil photographique, on détermine à partir des données de détection correspondantes une position et une direction (12) de vision de cette partie (4, 15) du dispositif (3, 4, 15) de détection par rapport au point (17) de référence.

9. Procédé (1) suivant l'une des revendications 2 à 8, **caractérisé en ce que**, dans la représentation stéréoscopique, on adapte automatiquement l'image (19) d'appareil photographique dans une partie (24), dans laquelle celle-ci est à chevauchement avec l'image (20) de superposition, par diaphragmage, mise en grisé, floutage et/ou représentation partiellement transparente.

10. Procédé (1) suivant l'une des revendications 2 à 9, **caractérisé en ce que** l'on enregistre l'image (19) d'appareil photographique sous la forme d'une image en 2D et, par la fixation de sa position en profondeur stéréoscopique sur la position en profondeur stéréoscopique du point (17) de référence dans la représentation stéréoscopique, on la représente complètement et unitairement dans cette position en profondeur stéréoscopique.

11. Procédé (1) suivant l'une des revendications 2 à 9, **caractérisé en ce que** l'on enregistre l'image (19) d'appareil photographique sous la forme d'une image en 2D et, avant la production de la représentation stéréoscopique, on la transforme au moyen d'un procédé de conversion de 2D en 3D et on produit ensuite la représentation stéréoscopique à partir de l'image d'appareil photographique transformée et de l'image (20) de superposition, dans lequel pour la fixation de la position en profondeur stéréoscopique de l'image d'appareil photographique transformée, on fixe une région partielle comprenant le point (17) de référence de l'image d'appareil photographique transformée sur la position en profondeur stéréoscopique du point (17) de référence.

12. Procédé (1) suivant la revendication 11, **caractérisé en ce que** l'on enregistre d'abord un enregistrement en 3D de la partie (10, 14) opaque optiquement et on met en coïncidence un modèle en 3D, qui en est produit, de la partie opaque optiquement avec l'image d'appareil photographique en 2D et on l'utilise comme base pour la transformation de l'image d'appareil photographique en 2D.

13. Programme d'ordinateur, qui code les stades d'un procédé (1) suivant l'une des revendications 2 à 12 et qui est conçu pour être chargé dans une mémoire (8) de données d'un système (2) d'imagerie afin d'exécuter les stades du procédé.

14. Mémoire (8) de données d'un système (2) d'imagerie, dans lequel il est mis en mémoire dans la mémoire (8) de données un code de programme, qui comprend au moins un programme d'ordinateur suivant la revendication 13.
